# EUROPEAN PATENT SPECIFICATION

(11) **EP 1 727 571 B1**
(45) Date of publication and mention of the grant of the patent: **22.02.2012**
(21) Application number: 05722334.9
(22) Date of filing: 18.03.2005
(51) Int. Cl.: A61L 27/44, A61L 27/58

(54) **METHOD FOR OBTAINING GRADED PORE STRUCTURE IN SCAFFOLDS FOR TISSUES AND BONE**
VERFAHREN ZUR GEWINNUNG EINER GRADIERTEN PORENSTRUKTUR IN GERÜSTEN FÜR GEWEBE UND KNOCHEN
PROCEDE PERMETTANT D'OBTENIR UNE STRUCTURE POREUSE CALIBREE DANS DES ECHAFAUDAGES DESTINES A DES TISSUS ET DES OS

(30) Priority: 22.03.2004 SG 200401544
(43) Date of publication of application: 06.12.2006
(73) Proprietor: Agency for Science, Technology and Research, Singapore 138632 (SG); Bio-Scaffold International Pte. Ltd., Singapore 117525 (SG)
(72) Inventor: MARGAM, Chandrasekaran, Singapore 650103 (SG); ZHANG, Su, Xia, Singapore 648352 (SG); TAY, Bee, Yen, Singapore 578713 (SG)
(74) Representative: Viering, Jentschura & Partner
(86) International application number: PCT/SG2005/000087
(87) International publication number: WO 2005/089827

(56) References cited:
- EP-A1- 1 216 717
- EP-A1- 1 216 717
- WO-A1-02/30481
- WO-A1-02/060508
- WO-A1-02/060508
- WO-A2-99/11297
- WO-A2-02/051463
- WO-A2-03/070186
- US-A1- 2002 004 573
- US-A1- 2002 062 154
- US-A1- 2002 138 154
- US-A1- 2002 183 858
- US-A1- 2003 114 936
- US-B1- 6 255 359
- THOMSON R C; ET AL: "FABRICATION OF BIODEGRADABLE POLYMER SCAFFOLDS TO ENGINEER TRABECULAR BONE" JOURNAL OF BIOMATERIALS SCIENCE. POLYMER EDITION, VSP, UTRECHT, vol. 7, no. 1, 1 January 1995 (1995-01-01) , pages 23-38, XP009037155 ISSN: 0920-5063
- MIKOS A G; TEMENOFF J S: "FORMATION OF HIGHLY POROUS BIODEGRADABEL SCAFFOLDS FOR TISSUE ENGINEERING" EJB ELECTRONIC JOURNAL OF BIOTECHNOLOGY, CONICYT, SANTIAGO, CL, vol. 3, no. 2, 15 August 2000 (2000-08-15) , pages 1-12, XP001205451 ISSN: 0717-3458
- DATABASE WPI Week 200357, 12 June 2003 Derwent Publications Ltd., London, GB; Class A96, AN 2003-607789, XP008110635 & WO 03 048241 A1 (NICEM LTD)

## Description

### Field of the Invention

This invention relates to a method for obtaining a graded pore structure in scaffolds for tissue and bone as defined in the claims. Also described herein are scaffolds for tissue and bone with a graded pore structure. Particularly, though not exclusively, the graded pore structure provides the initial structural strength and allows tissue growth with controlled degradation.

### Background to the Invention

Tissue engineering techniques generally require the use of a temporary scaffold as a three-dimensional template for initial cell attachment and subsequent tissue formation. The ability of the scaffold to be metabolised by the body allows it to be gradually replaced by new cells to form functional tissue.

Many conventional techniques ranging from fibre bonding, solvent casting, and particulate leaching have been developed to design and fabricate scaffolds. Rapid prototyping technology has also enabled the design and fabrication of scaffolds using computer control technology.

Biodegradable polymers have been attractive candidates for scaffolding materials because they degrade as the new tissues are formed, eventually leaving nothing foreign to the body. A few techniques such as salt leaching, fibrous fabric processing, gas foaming, emulsion freeze-drying, three-dimensional printing, and phase separation have been developed to generate highly porous polymer scaffolds for tissues engineering. However, to engineer clinically useful scaffolds for bones, tissues and organs is still a challenge.

A scaffold is expected to possess the following characteristics for use in tissue engineering:
(i) a three-dimensional structure that is porous with an interconnected pore network for cell/tissue growth and flow transport of nutrients and metabolic waste;
(ii) should be either biodegradable or bioresorbable with a controllable degradation and resorption rate to match cell/tissue growth in vitro and/or in vivo;
(iii) surface chemistry should be conducive for cell attachment, proliferation, and differentiation;
(iv) adequate mechanical properties to match those of the tissues at the site of implantation; and
(v) should be easily processed to form a variety of shapes and sizes.

In addition to the above properties, the scaffold should be able to perform guided tissue regeneration. This requires functionally graded properties across the three dimensional network. Of this pore size, porosity, and surface area (surface-to-volume ratio) are widely recognized as important parameters for a scaffold for tissue engineering. Other architectural features such as pore shape, pore wall morphology, and interconnectivity between pores of the scaffolding material, are also suggested to be important for cell seeding, migration, growth, mass transport, gene expression, and new tissue formation in three dimensions.

Four types of biomaterials have been experimentally and/or clinically studied as scaffold material for tissue engineering applications:
(i) synthetic organic materials, such as, for example, aliphatic polyesters, polyethylene glycol:
(ii) synthetic inorganic materials, such as, for example, hydroxyapatite, tricalciumphosphate, plaster of Paris, glass ceramics;
(iii) organic materials of natural origin, such as, for example, collagen, fibrin glue, hyualuronic acid; and
(iv) inorganic material of natural origin, such as, for example, coralline hydroxyapatite.

In general, the scaffold should be fabricated from a highly biocompatible material that does not generate immunorejection upon being implanted in the human body.

Current technological restrictions with process and equipment pose serious limitations in achieving a scaffold design with the desired interconnectivity in pores. A simple example of process limitation is the use of stereolithography. This needs photo-curable polymers to obtain the required structure, and has limitations on the maximum strength attainable. Three-dimensional printing technologies depend on binder formulation for obtaining the required structure. Methodologies such as salt leaching and phase separation suffer from limitations in creating the necessary interconnectivity of pores. For example, US 6,255,359 B1 discloses a polymeric scaffold with a porous gradient obtained by a particular distribution of pore-forming agents which are mixed together with the polymer solution before scaffold formation. After scaffold forming the pore-forming agent is entirely removed via leaching.

Current scaffolds for tissue engineering use either a rapid prototyping technology or gas foaming/solvent leaching methodology for obtaining a porous structure.

### Summary of the Invention

Described herein is a scaffold for tissue regeneration or bone growth, the scaffold being fabricated from at least two polymers according to the method of the present invention. The polymers are of differing rates of bio-degradabillty. A first polymer of the at least two polymers may able to be leached (or removed) by a solvent, and all other polymers of the at least two polymers may be inert to the solvent or may have a lower dissolution rate in the solvent. The scaffold may have a graded porosity with high porosity at a surface of the scaffold, and low porosity at a core of the scaffold.

Also described herein is a scaffold for tissue regeneration or bone growth; the scaffold being fabricated from at least two polymers. A first polymer of the at least two polymers is able to be leached (or removed) by a solvent, and all other polymers of the at least two polymers are inert to the solvent or may have a lower dissolution rate in the solvent.

Further described herein is a scaffold for tissue regeneration, or bone growth; the scaffold having a graded porosity with high porosity at a surface of the scaffold, and low porosity at a core of the scaffold. The scaffold may be fabricated from at least two polymers of differing rates of biodegradability. Additionally or alternatively, the scaffold may be fabricated from at least two polymers, a first polymer of the at least two polymers being able to be leached (or removed) by a solvent, and all other polymers of the at least two polymers being inert to the solvent or may have a lower dissolution rate in the solvent.

The invention provides a method of fabrication of a scaffold having a graded porosity with a high porosity at a surface of the scaffold and low porosity at a core of the scaffold for at least one of: tissue regeneration and bone growth; the method comprising:
(a) blending at least two polymers to form a polymer blend;
(b) forming the scaffold from the polymer blend;
(c) leaching the scaffold in a bath of a solvent to remove a first polymer of the at least two polymers, all other polymers of the at least two polymers being selected from the group consisting of: inert to the solvent, and having a lower dissolution rate in the solvent than the first polymer, wherein leaching of the first polymer is controlled so that removal of the first polymer occurs to a greater extent at the surface of the scaffold, and to a lesser extent at the core of the scaffold; and wherein leaching is controlled by a method selected from the group consisting of controlling the time of immersion of the scaffold in the bath of the solvent; and controlling agitation of the solvent in the bath of the solvent.

A scaffold described herein is manufactured according to the method referred to in the claims. Also described herein is a method of fabrication of a scaffold for tissue regeneration, or bone growth; the method comprising:
(a) blending at least two polymers to form a polymer blend;
(b) forming the scaffold from the polymer blend;
(c) leaching the scaffold using a solvent to remove a first polymer of the at least two polymers, all other polymers of the at least two polymers being inert to the solvent or may have a lower dissolution rate in the solvent.

The polymers may be milled prior to blending. All polymers of the at least two polymers may have a different rate of biodegradability. The leaching may be for only a part of the scaffold. That part may be at or adjacent a surface of the scaffold, but not the core of the scaffold. Preferably, the first polymer has faster rate of bio-degradability.

Forming may be by at least one method selected from: compression moulding, injection moulding, rapid prototyping, and three-dimensional printing. Compression moulding may be at a pressure in the range 0 to 20 Mpa, and at a temperature in the range 25°C to 80°C. Leaching may be in an ultrasonic bath of the solvent, the solvent preferably at a temperature in the range 25°C to 50°C, frequencies preferably being in the range 1KHz to 40KHz, and exposure time preferably being in the range 5 minutes to 120 minutes. The at least two polymers may be milled in a cryogenic mill and blended or be milled and blended in a cryogenic mill to form a preferred particle size in the range 20 to 500µm.

Milling may be for a cycle that may be dependant upon a desired particle size for the polymers, and the type of the polymers. Milling may be at a frequency in the range 15 to 30 cycles of one minute each, and the impaction rate may be 15 impacts/second.

At least two polymers may be purely synthetic polymers such as polyglycolide, poly L-lactide, poly DL-lactide, polylactide co-glycolide, polycaprolactone, or polyhydroxybutrate; or a blend of synthetic and natural polymers, or natural polymers. The solvent may be acetone, dichloromethane, hexfluoroisopropanol, chloroform, or alcohol or similar organic solvents.

There may be two polymers in a ratio in the range 60:40 to 30:70.

### Brief Description of the Drawings

In order that the invention may be readily understood and put into practical effect, there shall now be described by way of non-limitative example only preferred embodiments of the present invention, the description being with reference to the accompanying illustrative drawings in which:
Figure 1 is an illustration of a scaffold described herein,
Figure 2 is a vertical cross-sectional view of the scaffold of Figure 1;
Figure 3 is cofocal images of two samples after leaching by different solvents;
Figure 4 is optical images of samples before leaching, and after leaching by different solvents;
Figure 5 shows to graphs being porosity profiles of a sample; and
Figure 6 is an illustration of one embodiment of the production of a scaffold.

### Detailed Description of Preferred Embodiments

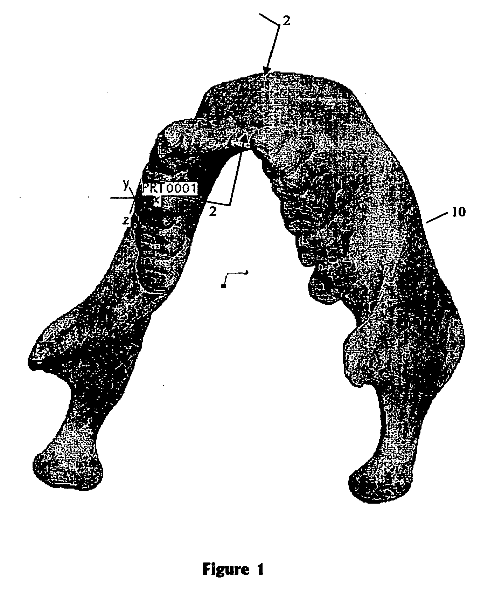

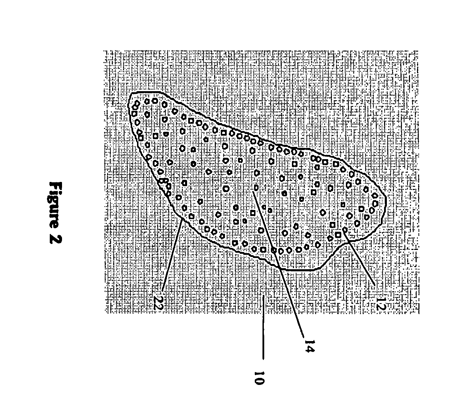

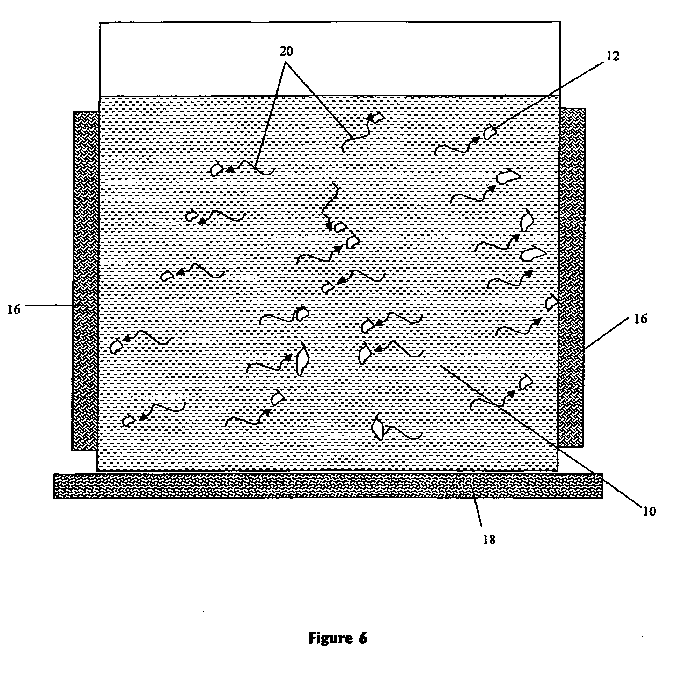

To refer to Figures 1, 2 and 6, there is shown an example of a scaffold 10 for tissue regeneration and/or bone growth.

In order to allow the scaffold 10 to closely mimic the tissue and to improve the quality of tissue regeneration/bone growth that occurs within the implant, the scaffold 10 has graded properties including graded porosity. This helps in obtaining optimum in-growth of different tissues and cells and enhances the ability of the implant to withstand varying mechanical loads at specific regions of the implant. The graded structure of pores 12 assists in delivering growth agents in a controlled manner. It would therefore assist in having controlled and guided osteogenesis and angiogenesis. A polymer blend is used, with the different polymers having different degradation rates that preferably correspond to the formation of the tissue, bone and blood vessels. This provides sufficient support and strength to the scaffold to assist the regeneration processes.

The pores 12 are graded so that as the growth initiates from the surface, the core 14 of the scaffold 10 provides the necessary support and retardation of tissue growth thus allowing the angiogenesis to take place in the core.

In one form, two different polymers were used, although any required number of polymers may be used depending on the required characteristics of the scaffold to be constructed. For example, three or four different polymers may be used.

If two polymers are used, the ratio of the two polymers used may be in the range 60:40 to 30:70. Again, the exact ratio will depend on the required characteristics of the scaffold to be constructed. The polymers may be natural or synthetic polymers and may include, but are not limited to: polyglycolide, polylactide (including poly DL-lactide and poly L-lactide), polylactide co-glydide, polycaprolactone, and polyhydroxylutrate.

To fabricate the scaffold 10, the polymers are selected and, in the correct ratio, milled to powder and blended to form a polymeric blend. The blended powder is sieved to size and the blend is used to fabricate the scaffold. The scaffold is then subjected to secondary processing. Milling may be in a cryogenic mill, and may include blending.

The secondary processing is controlled leaching of one of the polymers in the polymeric blend. Leaching may be by use of a solvent 20, preferably an organic solvent, to enhance the surface porosity, but with reduced concentration of pores 12 at the core 14 of the scaffold 10. By using a solvent that reacts with only a first of the polymers but not the other polymer(s), or has limited or slower reactivity with the other polymer(s), mainly the first polymer is removed by the leaching. This requires all other polymers to be either inert, have a lower rate of reaction to the solvent, or have a lower dissolution rate to the solvent. Leaching may be enhanced by controlling the temperature of the leaching solvent by use of heaters 16 and/or by agitation such as by ultrasonic agitation using ultrasonic table 18 or in a ultrasonicator.

By having different ratios of the polymers in different regions of the scaffold, different porosity will result from the leaching. Alternatively or additionally, the leaching process may be controlled so that the leaching will be of a greater extent at the surface of the scaffold, and of a lesser extent at the core 14 of the scaffold 10. This may be by controlling the rate of immersion of the scaffold 10 in a bath of the solvent, controlling the time of immersion so that the solvent will not fully complete the dissolving of the first polymer nearer the core, or otherwise. As leaching will start at the surface of the scaffold 10 and progress towards the core 14 through the pores 12 created by leaching, by controlling the duration, temperature and agitation of the solvent bath, the core 14 may be only partially leached, or there may be no leaching at the core 14 of the scaffold 10. In this way the pores 12 will reduce in number, size and connectivity from the surface 22 of the scaffold 10 to the core 14 of the scaffold 10.

Where the porosity is higher the rate of biodegradation will be faster, and where the porosity is lower, the slower the rate of degradation. By having a higher porosity at the surface 22, where tissue/bone regeneration/growth is earlier and fastest, the scaffold 10 will biodegrade as the regeneration/growth takes place. With lower porosity at the core 14, the scaffold will retain structural strength as the tissue/bone regenerates/grows such that the combined strength remains relatively uniform.

By having at least two different polymers with different rates of bio degradability, this can be enhanced. Also, the faster degrading polymer will progressively degrade towards the core 14 to thus enhance tissue/bone regeneration growth. In one example the polymer that is leached by the solvent have the faster/fastest rate of bio-degradabitity.

In one example, polyglycolide granules, poly L-lactide granules and polylactide co-glycolide granules were milled at a frequency of 15 to 30 cycles each for one minute. The exact cycle used depends on one or more of: the polymers used, particle size, and the glass transition temperature of the polymers. In one example the particle size is in the range 20-500 µm. An impact rate of 15 impacts/second was used.

The organic solvents used may be selected from: acetone, dichloromethane, hexfluoroisopropanol, chloroform, alcohol, or any other suitable organic solvent for selectively dissolving one of the polymers, and to which the other polymers are inert, or to which they have a lower dissolution rate.

Fabrication of the scaffold 10 may be by methods such as, for example, compression moulding, injection moulding, or rapid prototyping such as three-dimensional printing of the scaffold using a suitable binder system. For example, compression moulding at a pressure of 0 to 20 MPa and a temperature in the range 25 to 80°C may be used.

The solvent may be in a bath into which the scaffold is placed. The temperature of the solvent in the bath may be in the range 25°C to 50°C. The bath may be an ultrasonic bath using a frequency in the range 1KHz to 40KHz. The time of exposure of the scaffold in the bath may be in the range 5 minutes to 120 minutes.

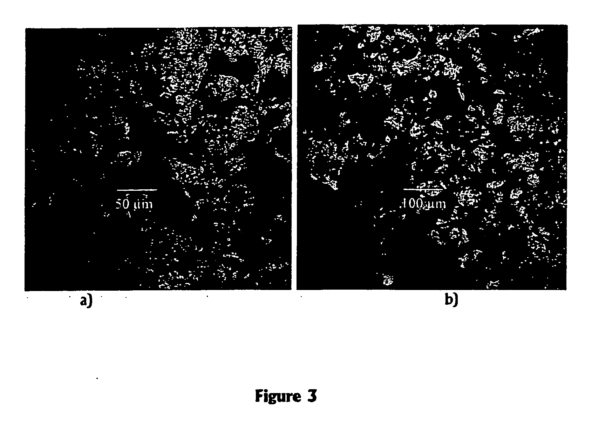

As is clear from Figure 3, there is good interconnectivity between the pores, and a good three-dimensional network of pores. The polymers used were:
(a) polyglycolide and polylactide co-glycolide in a ratio of 50:50 as leached in dichloromethane; and
(b) polyglycolide and polyhydroxybutrate in a ratio of 50:50 as leached in acetone.

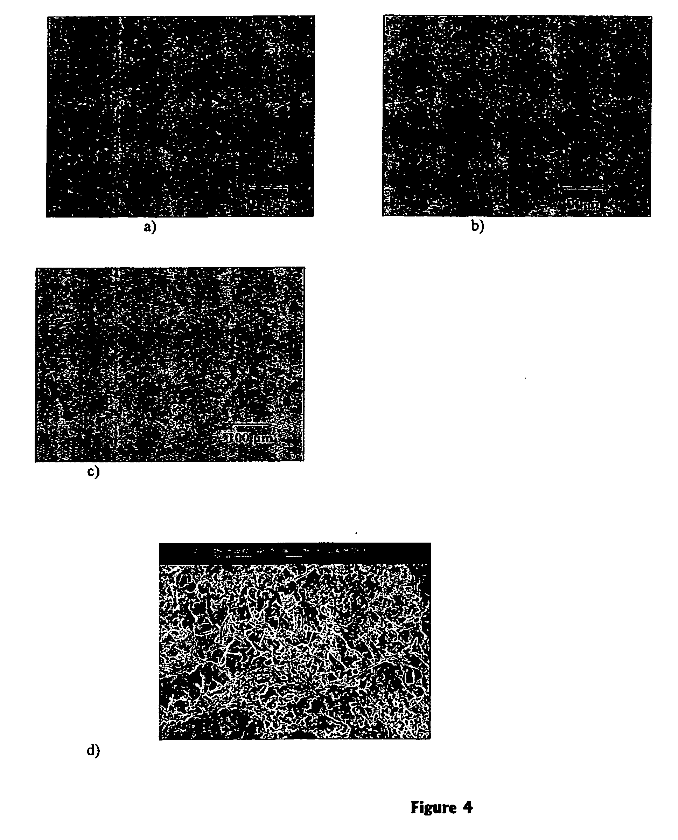

Figure 4 shows the sample (a) above:
(a) before [(a)];
   and after leaching in:
(b) dichloromethane; and
(c) acetone;
and shows a uniform distribution of surface pores, and interconnectivity between them.

Figure 4(d) shows porosity distribution in the core with micropores of a pore size in the range of 10 µm or less.

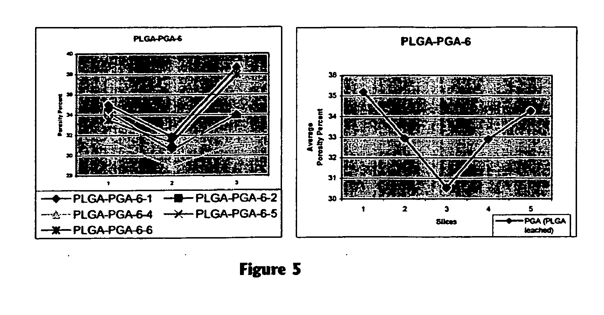

Figure 5 shows two porosity profiles obtained in a poly L-lactide/polyglycolide sample after subjecting it to secondary processing. The porosity measurement was by use of a profilometer which had a resolution of pore size greater than 10 µm.

## Claims

1. A method of fabrication of a scaffold having a graded porosity with a high porosity at a surface of the scaffold and low porosity at a core of the scaffold for at least one of:
tissue regeneration and bone growth; the method comprising:
(a) blending at least two polymers to form a polymer blend;
(b) forming the scaffold from the polymer blend;
(c) leaching the scaffold in a bath of a solvent to remove a first polymer of the at least two polymers, all other polymers of the at least two polymers being selected from the group consisting of: inert to the solvent, and having a lower dissolution rate in the solvent than the first polymer,
wherein leaching of the first polymer is controlled so that removal of the first polymer occurs to a greater extent at the surface of the scaffold, and to a lesser extent at the core of the scaffold; and wherein leaching is controlled by a method selected from the group consisting of controlling the time of immersion of the scaffold in the bath of the solvent; and controlling agitation of the solvent in the bath of the solvent.

2. The method as claimed in claim 1, wherein all polymers of the at least two polymers all have a different rate of biodegradability.

3. The method as claimed in any one of claims 1 to 2, wherein the at least two polymers are selected from the group consisting of: natural polymers, a blend of natural polymers and synthetic polymers, synthetic polymers, polyglycolide, polylactide, poly L-lactide, poly DL-lactide, polylactide co-glycolide, poly caprolactone, and polyhydroxybutrate.

4. The method as claimed in any one of claims 1 to 3, wherein the solvent is selected from the group consisting of: acetone, dichloromethane, hexfluoroisopropanol, chloroform, and alcohol.

5. The method as claimed in any one of claims 1 to 4, wherein the forming is by at least one method selected from the group consisting of: compression moulding, injection molding, rapid prototyping, and three dimensional printing.

6. The method as claimed in claim 5, wherein compression moulding is at a pressure in the range 0 to 20 Mpa, and at a temperature in the range 25°C to 80°C.

7. The method as claimed in claim 2, wherein the first polymer has a faster rate of bio-degradability.

8. The method as claimed in any one of claims 1 to 7, wherein agitation is ultrasonic agitation.

9. The method as claimed in claim 8, wherein the solvent is at a temperature in the range 25 °C to 50°C, frequencies being in the range 1KHz to 40KHz, and exposure time being in the range 5 minutes to 120 minutes.

10. The method as claimed in any one of claims 1 to 8, wherein the at least two polymers are milled prior to blending, milling and blending being in a cryogenic mill to form a particle size in the range 20 to 500 µm.

11. The method as claimed in claim 10, wherein the milling is at a cycle dependent upon at least one of: the type of the at least two polymers, and a desired particle size of the at least two polymers.

12. The method as claimed in claim 10 or 11, wherein milling is at a frequency in the range 15 to 30 cycles of one minute each.

13. The method as claimed in any one of claims 10 to 12, wherein during milling, an impaction rate is 15 impacts/second.

## Patentansprüche

1. Ein Verfahren zur Herstellung einer Gerüststruktur, die eine gestaffelte Porosität mit einer hohen Porosität an der Oberfläche der Gerüststruktur und einer niedrigen Porosität im Kern der Gerüststruktur besitzt, für mindestens eines von:
Geweberegeneration und Knochenwachstum; wobei das Verfahren umfasst:
(a) Vermischen von mindestens zwei Polymeren, um eine Polymermischung zu bilden;
(b) Ausbilden der Gerüststruktur aus der Polymermischung;
(c) Extrahieren der Gerüststruktur in einem Lösungsmittelbad, um ein erstes Polymer von den mindestens zwei Polymeren zu entfernen, wobei alle anderen Polymere von den mindestens zwei Polymeren ausgewählt werden aus der Gruppe bestehend aus: Polymere, die bezogen auf das Lösungsmittel inert sind, und Polymere, die eine niedrigere Auflösungsrate in dem Lösungsmittel besitzend als das erste Polymer,
wobei das Extrahieren des ersten Polymers kontrolliert wird, so dass das Entfernen des ersten Polymers in einem größerem Ausmaß an der Oberfläche der Gerüststruktur erfolgt und zu einem geringeren Ausmaß in dem Kern der Gerüststruktur; und wobei das Extrahieren durch ein Verfahren kontrolliert wird, das ausgewählt wird aus der Gruppe bestehend aus Kontrollieren der Zeit des Eintauchens der Gerüststruktur in dem Lösungsmittelbad; und Kontrollieren des Schüttelns des Lösungsmittels in dem Lösungsmittelbad.

2. Das Verfahren, wie in Anspruch 1 beansprucht, wobei alle Polymere der mindestens zwei Polymere eine unterschiedliche Bio-Abbaubarkeitsrate besitzen.

3. Das Verfahren, wie in einem der Ansprüche 1 bis 2 beansprucht, wobei die mindestens zwei Polymere ausgewählt werden aus der Gruppe bestehend aus: natürlichen Polymeren, eine Mischung aus natürlichen Polymeren und synthetischen Polymeren, synthetischen Polymeren, Polyglycolid, Polylactid, Poly-L-Lactid, Poly-DL-Lactid, Polylactid-co-Glycolid, Polycaprolacton und Polyhydroxybutyrat.

4. Das Verfahren, wie in einem der Ansprüche 1 bis 3 beansprucht, wobei das Lösungsmittel ausgewählt wird aus der Gruppe bestehend aus: Aceton, Dichlormethan, Hexafluoroisopropanol, Chloroform und Alkohol.

5. Das Verfahren, wie in einem der Ansprüche 1 bis 4 beansprucht, wobei das Ausbilden durch mindestens ein Verfahren erfolgt, das ausgewählt wird aus der Gruppe bestehend aus:
Pressformen, Spritzformen, schnellem Prototypisieren und dreidimensionalem Drucken.

6. Das Verfahren, wie in Anspruch 5 beansprucht, wobei das Pressformen bei einem Druck im Bereich von 0 bis 20 MPa und bei einer Temperatur im Bereich von 25 °C bis 80 °C erfolgt.

7. Das Verfahren, wie in Anspruch 2 beansprucht, wobei das erste Polymer eine schnellere Bio-Abbaubarkeitsrate besitzt.

8. Das Verfahren, wie in einem der Ansprüche 1 bis 7 beansprucht, wobei das Schütteln Ultraschall-Schütteln ist.

9. Das Verfahren, wie in Anspruch 8 beansprucht, wobei das Lösungsmittel eine Temperatur in dem Bereich von 25 °C bis 50 °C besitzt, die Frequenzen in dem Bereich von 1 kHz bis 40 kHz liegen und die Behandlungszeit in dem Bereich von 5 Minuten bis 120 Minuten liegt.

10. Das Verfahren, wie in einem der Ansprüche 1 bis 8 beansprucht, wobei die mindestens zwei Polymere vor dem Vermischen gemahlen werden, wobei das Mahlen und Mischen in einer kryogenen Mühle erfolgt, um eine Partikelgröße in dem Bereich von 20 bis 500 µm zu erhalten.

11. Das Verfahren, wie in Anspruch 10 beansprucht, wobei das Mahlen zyklisch erfolgt, in Abhängigkeit von mindestens einem von: dem Typ der mindestens zwei Polymere und der gewünschten Partikelgröße der mindestens zwei Polymere.

12. Das Verfahren, wie in Anspruch 10 oder 11 beansprucht, wobei das Mahlen mit einer Frequenz im Bereich 15 bis 30 Zyklen für jeweils 1 Minute erfolgt.

13. Das Verfahren, wie in einem der Ansprüche 10 bis 12 beansprucht, wobei während des Mahlens die Stoßrate 15 Stöße/Sekunde beträgt.

## Revendications

1. Procédé de fabrication d'un échafaudage ayant une porosité calibrée avec une porosité élevée à la surface de l'échafaudage et une faible porosité au coeur de l'échafaudage destiné à au moins l'une parmi :
la régénération tissulaire et la croissance osseuse ; le procédé consistant à :
(a) mélanger au moins deux polymères pour former un mélange de polymères ;
(b) former l'échafaudage à partir du mélange de polymères ;
(c) plonger l'échafaudage dans un bain d'un solvant pour éliminer un premier polymère parmi les au moins deux polymères, tous les autres polymères parmi les au moins deux polymères étant choisis parmi le groupe consistant en : inerte par rapport au solvant, et ayant une vitesse de dissolution plus lente dans le solvant que dans le premier polymère,
où l'immersion du premier polymère est contrôlée de sorte que l'élimination du premier polymère ait lieu à un degré plus grand à la surface de l'échafaudage, et à un degré moindre au coeur de l'échafaudage ; et l'immersion est contrôlée par un procédé choisi parmi le groupe consistant en contrôle du temps d'immersion de l'échafaudage dans le bain du solvant ; et contrôle de l'agitation du solvant dans le bain du solvant.

2. Procédé selon la revendication 1, dans lequel l'ensemble des polymères des au moins deux polymères ont tous une vitesse de biodégradabilité différente.

3. Procédé selon l'une quelconque des revendications 1 à 2, dans lequel les au moins deux polymères sont choisis parmi le groupe consistant en : polymères naturels, un mélange de polymères naturels et de polymères synthétiques, polymères synthétiques, polyglycolide, polylactide, poly L-lactide, poly DL-lactide, polylactide co-glycolide, poly caprolactone, et polyhydroxybutyrate.

4. Procédé selon l'une quelconque des revendications 1 à 3, dans lequel le solvant est choisi parmi le groupe consistant en: acétone, dichlorométhane, hexafluoroisopropanol, chloroforme, et alcool.

5. Procédé selon l'une quelconque des revendications 1 à 4, dans lequel la formation est réalisée par au moins un procédé choisi parmi le groupe consistant en : moulage par compression, moulage par injection, prototypage rapide, et impression en trois dimensions.

6. Procédé selon la revendication 5, dans lequel le moulage par compression est réalisé à une pression dans le domaine de 0 à 20 MPa, et à une température dans le domaine de 25° C à 80° C.

7. Procédé selon la revendication 2, dans lequel le premier polymère présente une vitesse plus rapide de biodégradabilité.

8. Procédé selon l'une quelconque des revendications 1 à 7, dans lequel l'agitation est une agitation par ultrasons.

9. Procédé selon la revendication 8, dans lequel le solvant est à une température dans le domaine de 25° C à 50° C, les fréquences étant dans le domaine de 1 kHz à 40 kHz, et le temps d'exposition étant dans le domaine de 5 minutes à 120 minutes.

10. Procédé selon l'une quelconque des revendications 1 à 8, dans lequel les au moins deux polymères sont broyés avant d'être mélangés, le broyage et le mélange étant réalisés dans un broyeur cryogénique pour former une taille de particules dans le domaine de 20 à 500 µm.

11. Procédé selon la revendication 10, dans lequel le broyage est réalisé à un cycle dépendant de l'au moins un parmi : le type des au moins deux polymères, et une taille de particules désirée des au moins deux polymères.

12. Procédé selon la revendication 10 ou 11, dans lequel le broyage est réalisé à une fréquence dans le domaine de 15 à 30 cycles d'une minute chacun.

13. Procédé selon l'une quelconque des revendications 10 à 12, dans lequel durant le broyage, une vitesse d'impact est de 15 impacts/seconde.
